# EUROPEAN PATENT SPECIFICATION

(11) **EP 2 964 197 B1**
(45) Date of publication and mention of the grant of the patent: **25.03.2020**
(21) Application number: 14710738.7
(22) Date of filing: 28.02.2014
(51) Int. Cl.: A61K 9/14, A61K 9/16, A61K 9/20, A61K 31/426

(54) **SOLID COMPOSITIONS COMPRISING A GLUCOKINASE ACTIVATOR AND METHODS OF MAKING AND USING THE SAME**
FESTE ZUSAMMENSETZUNGEN MIT EINEM GLUCOKINASEAKTIVATOR UND VERFAHREN ZUR HERSTELLUNG UND VERWENDUNG DAVON
COMPOSITIONS SOLIDES COMPRENANT UN ACTIVATEUR DE GLUCOKINASE ET PROCÉDÉS DE FABRICATION ET D'UTILISATION ASSOCIÉS

(30) Priority: 04.03.2013 US 201361771969 P
(43) Date of publication of application: 13.01.2016
(73) Proprietor: vTv Therapeutics LLC, High Point, NC 27265 (US)
(72) Inventor: BENJAMIN, Eric, Jamestown, North Carolina 27282 (US); THORSTEINSSON, Thorsteinn, West Palm Beach, Florida 33411 (US); RAPURU, Siva Kumar, Jamestown, North Carolina 27282 (US)
(74) Representative: Elkington and Fife LLP
(86) International application number: PCT/US2014/019363
(87) International publication number: WO 2014/137799

(56) References cited:
- WO-A1-2011/149945
- US-B2- 7 872 139

## Description

### BACKGROUND OF THE INENTION

### FIELD OF THE INVENTION

The invention relates to solid pharmaceutical compositions comprising a glucokinase (GK) activator suitable for oral administration. The invention is also directed to methods of making and using such pharmaceutical compositions, and to solid dosage forms comprising such compositions.

### DESCRIPTION OF RELATED ART

Type 2 diabetes is a metabolic disorder where disease progression is typically characterized by one or more of the following symptoms: peripheral tissue insulin resistance, hyperglycemia, islet b-cell compensation, hyperinsulinemia, dyslipidemia, increased liver gluconeogenesis, and loss of b-cell mass and function. The pathophysiological consequences of aberrant glucose and lipid metabolism are toxicity to various organs, including, but not limited to, the kidneys, eyes, peripheral neurons, vasculature, and heart. Thus, there is a medical need for agents that may delay or prevent disease progression by improving glycemic control and helping maintain b-cell mass and function in diabetic patients.

Glucokinase (GK) is an enzyme that, among other things, facilitates phosphorylation of glucose to glucose-6-phosphate. In vertebrates, GK-mediated glucose phosphorylation typically occurs in cells in the liver, pancreas, gut, and brain. In each of these organs, GK may play a role in regulating carbohydrate metabolism by acting as a glucose sensor, triggering shifts in metabolism or cell function in response to rising or falling levels of blood-glucose.

Small-molecule GK activators are useful in treating type 2 diabetes because they can activate GK, and thereby indirectly reduce the body's demand for insulin. WO 2005/066145 describes novel compounds that are useful as GK activators, and that are therefore useful, among other things, for the treatment of type 2 diabetes. In particular, WO 2005/066145 describes the GK activator, {2-[3-cyclohexyl-3-(*trans*-4-propoxy-cyclohexyl)-ureido]-thiazol-5-ylsulfanyl}-acetic acid and pharmaceutically acceptable salts thereof (referred to collectively herein as "Urea Derivatives 1" or "UD1").

GK activators, such as UD1, may provide diabetic patients with improved glycemic control in comparison to traditional antidiabetic drugs, such as biguanides. GK activators may need to be administered up to several times a day over the course of years. Therefore, it is desirable to package the drug so as to enhance patient convenience. An oral dosage form is preferred, as the convenience of oral dosing generally improves patient compliance with a prescribed dosing regimen. Thus, there is a need for solid compositions comprising a GK activator, such as UD1, where the solid compositions have properties that facilitate their use in oral dosage forms. Such properties include, among other things, stability of the active ingredient within the composition and release of the active ingredient (e.g., in the stomach) so as to allow for effective absorption (e.g., in the upper part of the small intestine).

### SUMMARY OF THE INVENTION

The invention provides solid compositions comprising a glucokinase (GK) activator for use in the oral delivery of a drug.

In one aspect, the invention provides solid compositions comprising a GK activator and a pharmaceutically acceptable carrier, excipient, diluent, or a mixture thereof. In some embodiments, the solid composition comprises a GK activator in the form of a free acid. In some embodiments, including embodiments where a GK activator is in the form of a free acid, the solid composition further comprises a water-soluble surfactant. In some further embodiments, the solid composition comprises a GK activator, a water-soluble surfactant, and a pharmaceutically acceptable basic excipient and/or a binder.

In another aspect, the invention provides methods of making a solid composition comprising a GK activator. Such methods comprise mixing a GK activator with one or more additional ingredients in the presence of a solvent, and removing the solvent from the mixture. In some embodiments, the removing step comprises spray drying. In some further embodiments, the removing step comprises drying within a heated environment (e.g., within a fluid bed or within a tray).

In another aspect, the invention provides methods of using a solid composition that comprises a GK activator and a pharmaceutically acceptable carrier, excipient, diluent, or a mixture thereof. In some embodiments, the methods include, but are not limited to, one or more of the following: methods of treating type 2 diabetes, methods of treating type 1 diabetes, methods of improving glycemic control, methods of lowering blood-glucose, methods of enhancing phosphorylation of glucose, methods of improving insulin sensitivity, and the like.

In another aspect, the invention provides solid dosage forms comprising a solid composition comprising a GK activator and a pharmaceutically acceptable carrier, excipient, diluent, or a mixture thereof. In some embodiments, the solid dosage form is a capsule. In some embodiments, the solid dosage form is a tablet. In other embodiments, the solid dosage form is a powder (e.g., suspended within a liquid, packaged within a sachet, etc.). In yet other embodiments, the solid dosage form is encapsulated, or microencapsulated, or nanoencapsulated in a suitable pharmaceutical coating material or matrix material, where such coating materials or matrix materials can include, but are not limited to, sustained-release materials, controlled-release materials, enteric-release materials, rapid-dissolving materials, and the like.

### BRIEF DESCRIPTION OF DRAWINGS

Figure 1 shows the diffractogram from a PXRD analysis of a sample containing unmicronized crystalline {2-[3-cyclohexyl-3-(*trans-*4-propoxy-cyclohexyl)-ureido]-thiazol-5-ylsulfanyl}-acetic acid, collected using Cu-Ka radiation.
Figure 2 shows the diffractogram from a PXRD analysis of a sample containing micronized crystalline {2-[3-cyclohexyl-3-(*trans*-4-propoxy-cyclohexyl)-ureido]-thiazol-5-ylsulfanyl}-acetic acid, collected using Cu-Ka radiation.

### DETAILED DESCRIPTION

Patients with type 2 diabetes may exhibit a decreasing ability of their pancreas to secrete sufficient insulin to control post-prandial blood-glucose levels. Initially, type 2 diabetics may be able to control progression of the disease by following dietary restrictions, such as consuming foods having a low glycemic index. But as the disease progresses, diet alone is insufficient to control blood-glucose levels. Thus, medical intervention becomes necessary. At this stage (or even in advance of this stage), physicians may prescribe an oral antidiabetic agent to aid in glycemic control. Common oral antidiabetic agents include sulfonylureas, such as glibenclamide, and biguanides, such as metformin.

These common antidiabetics often have undesirable side-effects in many patient populations, and often fail to provide desirable levels of glycemic control. Thus, scientists have continued to search for compounds that can replace or supplement the use of these common antidiabetics. Glucokinase (GK) activators represent one such class of compounds.

GK is an enzyme that, among other things, facilitates phosphorylation of glucose to glucose-6-phosphate. In vertebrates, GK-mediated phosphorylation generally occurs in cells in the liver, pancreas, gut, and brain. In each of these organs, GK can play a role in regulating carbohydrate metabolism by acting as a glucose sensor, triggering shifts in metabolism or cell function in response to rising and/or falling levels of blood-glucose.

Small-molecule GK activators are useful in treating type 2 diabetes because they can enhance the rate of glucose phosphorylation, and thereby reduce the amount of glucose in the blood. Therefore, GK activators lower the body's demand for insulin, especially following intake of food. In this way, GK activators provide an alternate treatment option for type 2 diabetics who otherwise may have difficulty achieving effective glycemic control.

Various GK activators are known. For example, {2-[3-cyclohexyl-3-(*trans*-4-propoxy-cyclohexyl)-ureido]-thiazol-5-ylsulfanyl}-acetic acid is a GK activator. The preparation and pharmaceutical use of this molecule and pharmaceutically acceptable salts thereof are described in WO 2005/066145.

The therapeutic half lives of GK activators may vary from compound to compound. In general, however, it is expected that one would administer such drugs up to several times a day. Due to this frequency of administration, it may be convenient to administer the GK activator orally. Thus, the present invention is directed to novel solid compositions suitable for use in the oral delivery of a GK activator, in particular {2-[3-cyclohexyl-3-(*trans*-4-propoxy-cyclohexyl)-ureido]-thiazol-5-ylsulfanyl}-acetic acid and/or pharmaceutically acceptable salts thereof (UD1).

The preparation of such solid compositions presents a number of technical problems that may vary depending on the chemical and physical properties of the active compound. For example, the resulting formulation must have sufficient stability to withstand the pharmaceutical packaging process and to maintain compositional integrity during storage. Further, the composition must be capable of releasing the drug into the GI tract (e.g., the stomach) to allow for effective absorption (e.g., in the upper part of the small intestine).

It was discovered that {2-[3-cyclohexyl-3-(*trans*-4-propoxy-cyclohexyl)-ureido]-thiazol-5-ylsulfanyl}-acetic acid, as a free acid (referred to as "UD1-FA") has greater stability relative to some of its pharmaceutically acceptable salts. Because increased stability may lead to enhanced shelf life and may reduce difficulties in handling and packaging, it is desirable to have a solid composition that uses UD1-FA. In addition, the solid composition must be such that it releases the GK activator into solution within the stomach and/or the upper part of the small intestine. Otherwise, absorption may not occur to a substantial degree. Thus, in at least one aspect, the present invention is directed to solid compositions comprising UD1-FA, such that the UD1-FA in the solid composition is bioavailable in low-pH media. It was discovered that one could make such a solid composition by including a water-soluble surfactant in the solid composition with the UD1-FA.

### UD1-FA

UD1-FA may exist in both amorphous and crystalline forms. In various embodiments of the invention, UD1-FA can be present in either amorphous or crystalline forms, or as a mixture of amorphous and crystalline forms. As used herein, the term "amorphous," when used in reference to UD1-FA, refers to a solid-state form of UD1-FA characterized by the absence of any long-range order in the position of the atoms within the solid, where "long-range order" refers to order on a scale larger than about 5-10 times that of typical interatomic distances within the molecule. Furthermore, the term "crystalline," when used in reference to UD1-FA, refers to a solid-state form of UD1-FA characterized as having long-range order in the position of the atoms within the solid. Such crystalline solids need not consist exclusively of UD1-FA molecules, but may also incorporate solvent molecules into the crystalline lattice, so as to form solvates or hydrates of UD1-FA.

In some embodiments of the invention, the solid composition comprises UD1-FA in an amorphous form. Yet in some embodiments of the invention, the solid composition comprises UD1-FA in one or more crystalline forms. Further, in some embodiments, the solid composition comprises UD1-FA in an amorphous form and in one or more crystalline forms. The relative amounts of amorphous to crystalline forms in the solid composition will depend on various factors, including, but not limited to, the means of making the solid composition, the identity and relative amounts of other components in the solid composition, whether or not the solid composition has been packaged into a dosage form, and, if packaged into a finished dosage form, the nature of the packaging process and the dosage form. For example, the UD1-FA within the solid composition may have a lower degree of crystallinity following the addition of an amorphizing agent. In some embodiments, the solid composition comprises UD1-FA in one or more crystalline forms, where at least 50%, or at least 70%, or at least 90%, or at least 95%, or at least 99% of the UD1-FA in the solid composition is present in one or more crystalline forms.

In some embodiments where UD1-FA is present in the solid composition in a crystalline form, the crystalline form is substantially free of included solvate molecules. For example, in some such embodiments, the crystalline form of UD1 is at least about 95% by weight, or at least about 97% by weight, or at least about 99% by weight, or at least about 99.5% by weight UD1-FA.

The invention can employ UD1-FA having any particle size that is suitable for use in solid pharmaceutical compositions. In some embodiments, the solid composition comprises UD1-FA particles such that at least 80%, or at least 85%, or at least 90%, or at least 95% of the UD1-FA particles in the composition (based on the total weight of UD1-FA particles in the composition) have a particle size between 300 nm and 1 mm. In some further embodiments, the solid composition comprises UD1-FA particles such that at least 80%, or at least 85%, or at least 90%, or at least 95% of the UD1-FA particles in the composition (based on the total weight of UD1-FA particles in the composition) have a particle size between 500 nm and 500 µm. In some further embodiments, the solid composition comprises UD1-FA particles such that at least 80%, or at least 85%, or at least 90%, or at least 95% of the UD1-FA particles in the composition (based on the total weight of UD1-FA particles in the composition) have a particle size between 800 nm and 300 µm. In some further embodiments, the solid composition comprises UD1-FA particles such that at least 80%, or at least 85%, or at least 90%, or at least 95% of the UD1-FA particles in the composition (based on the total weight of UD1-FA particles in the composition) have a particle size between 1 µm and 100 µm. In some further embodiments, the solid composition comprises UD1-FA particles such that at least 90% of the UD1-FA particles in the composition (based on total weight of the UD1-FA particles in the composition) have a particle size greater than 0.1 µm. In some further embodiments, the solid composition comprises UD1-FA particles such that at least 95% of the UD1-FA particles in the composition (based on total weight of the UD1-FA particles in the composition) have a particle size less than 10 µm. In some further embodiments, the solid composition comprises UD1-FA particles such that at least 75% of the UD1-FA particles in the composition (based on total weight of the UD1-FA particles in the composition) have a particle size less than 5 µm. In some further embodiments, the solid composition comprises UD1-FA particles such that at least 95% of the UD1-FA particles in the composition (based on total weight of the UD1-FA particles in the composition) have a particle size between 0.1 µm and 100 µm, or 90% between 0.1 µm and 10 µm, or 85% between 0.4 µm and 6 µm.

In some embodiments, the solid composition comprises micronized UD1-FA, meaning that at least 80%, or at least 85%, or at least 90%, or at least 95% of the UD1-FA particles in the composition (based on the total weight of UD1-FA particles in the composition) have a particle size between 1 µm and 100 µm.

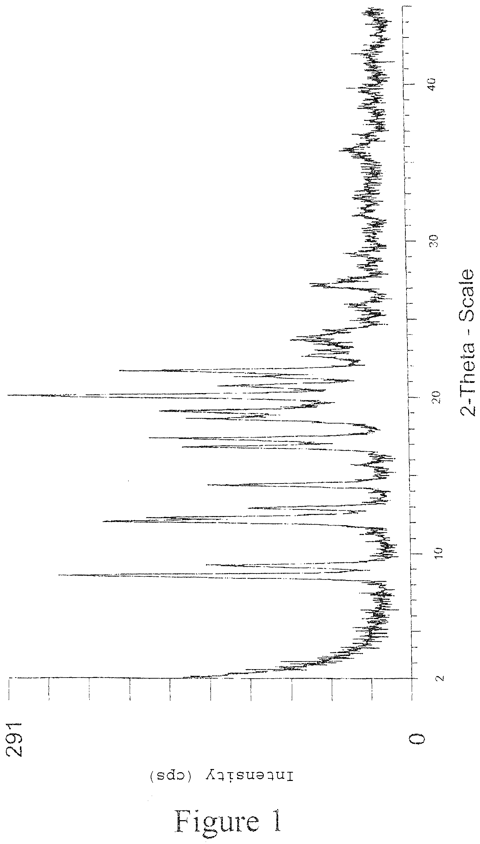

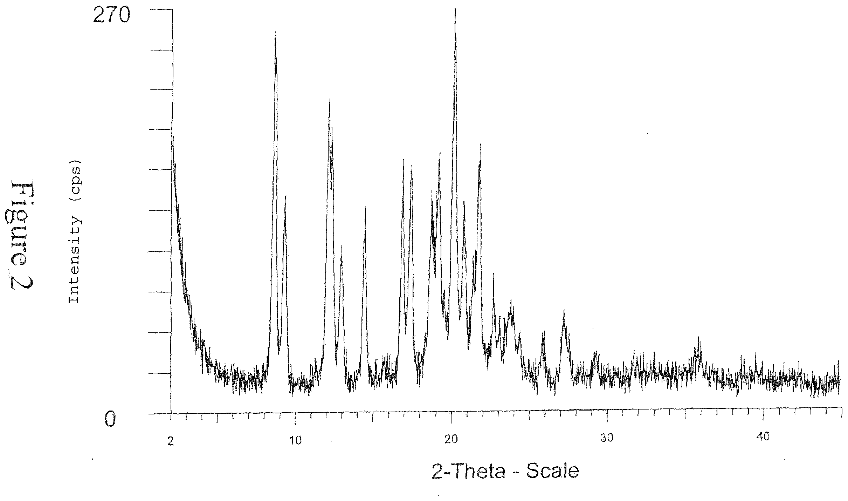

In some embodiments of the invention, the solid composition comprises a particular crystalline form of UD1-FA, referred to herein as "Form A". Figure 1 shows the diffractogram for the powder x-ray diffraction (Cu Kα, 25 °C) of an unmicronized sample of Form A. Table 1, below, shows the measured 2θ values and the corresponding d values for the diffractogram shown in Figure 1. Figure 2 shows the diffractogram for the powder x-ray diffraction (Cu Kα, 25 °C) of a micronized sample of Form A. Table 2, below, shows the measured 2θ values and the corresponding d values for the diffractogram shown in Figure 2. It has been determined that Form A of UD1-FA is particularly stable, and can therefore be used beneficially in products, such as oral therapeutics.

Based on the data shown in Tables 1 and 2, Form A can be described as a crystalline form of UD1-FA having several of the following interplanar spacings (in Å): 10.30, 9.54, 7.33, 7.20, 5.26, 5.10, 4.76, 4.64, 4.41, and/or 4.09. Depending on measurement conditions and the methods of preparing the sample, these values may vary by up to 0.02 Å, or up to 0.01 Å. It may not be necessary to employ all ten of the recited interplanar spacings to identify Form A. Therefore, in some embodiments, a smaller subset of the ten recited peaks can be employed to identify the presence of Form A. For example, when Form A is used in a solid composition with other materials, it may not be possible to distinguish some x-ray diffraction peaks of Form A from those of an excipient. In such instances, it can be sufficient to rely on a subset of the ten above-recited peaks to identify the presence of Form A in a solid composition of the invention. In some embodiments of the invention, the solid composition comprises Form A of UD1-FA. In some such embodiments, the solid composition comprises at least about 50%, or at least about 60%, or at least about 70%, or at least about 80%, or at least about 90%, or at least about 95% Form A of UD1-FA.

In embodiments of the invention, the solid composition comprises UD1 as a free acid (UD1-FA). The invention, however, does not exclude solid compositions that comprise an amount of a salt of UD1-FA. In some embodiments of the invention, the solid composition comprises UD1-FA and a salt of UD1-FA (e.g., a pharmaceutically acceptable salt of UD1-FA). In some such embodiments, the salt of UD1-FA is less than about 30%, or less than about 20%, or less than about 10%, or less than about 5%, or less than about 3%, or less than about 1%, or less than about 0.5%, or less than about 0.2% of the total weight of UD1 (as a free acid and a salt, collectively) present in the solid composition.

As used herein, the term "pharmaceutically acceptable salt," refers to salts of a free acid or a free base which are not biologically undesirable and are generally prepared by reacting the free base with a suitable organic or inorganic acid or by reacting the acid with a suitable organic or inorganic base. The term may be used in reference to any compound, including a GK activator (having a free acid or free base functionality). Representative salts include the following salts: Acetate, Benzenesulfonate, Benzoate, Bicarbonate, Bisulfate, Bitartrate, Borate, Bromide, Calcium Edetate, Camsylate, Carbonate, Chloride, Clavulanate, Citrate, Dihydrochloride, Edetate, Edisylate, Estolate, Esylate, Fumarate, Gluceptate, Gluconate, Glutamate, Glycollylarsanilate, Hexylresorcinate, Hydrabamine, Hydrobromide, Hydrochloride, Hydroxynaphthoate, Iodide, Isethionate, Lactate, Lactobionate, Laurate, Malate, Maleate, Mandelate, Mesylate, Methylbromide, Methylnitrate, Methylsulfate, Monopotassium Maleate, Mucate, Napsylate, Nitrate, N-methylglucamine, Oxalate, Pamoate (Embonate), Palmitate, Pantothenate, Phosphate/diphosphate, Polygalacturonate, Potassium, Salicylate, Sodium, Stearate, Subacetate, Succinate, Tannate, Tartrate, Teoclate, Tosylate, Triethiodide, Trimethylammonium and Valerate. When an acidic substituent is present (e.g., in a GK activator), such as -COOH, there can be formed the ammonium, morpholinium, sodium, potassium, barium, calcium salt, and the like, for use as the dosage form. When a basic group is present (e.g., in a GK activator), such as amino or a basic heteroaryl radical, such as pyridyl, an acidic salt, such as hydrochloride, hydrobromide, phosphate, sulfate, trifluoroacetate, trichloroacetate, acetate, oxalate, maleate, pyruvate, malonate, succinate, citrate, tartarate, fumarate, mandelate, benzoate, cinnamate, methanesulfonate, ethanesulfonate, picrate and the like, and include acids related to the pharmaceutically-acceptable salts listed in Stephen M. Berge, et al., Journal of Pharmaceutical Science, Vol. 66(1), pp. 1-19 (1977).

### Solid Compositions

In at least one aspect, the invention provides solid compositions comprising UD1 and a water-soluble surfactant. Such solid compositions can include UD1 according to any of the embodiments recited above (e.g., as UD1-FA).

As used herein, the term "solid composition" refers to a solid-state composition that is, or can be made into, a solid pharmaceutical dosage form. Thus, in some embodiments of the invention, the solid compositions are bulk powders comprising UD1-FA. In other embodiments, however, the solid compositions are in a dosage form suitable for oral administration to a subject, such as a capsule, microcapsule, nanocapsule, tablet, suspension, sachet, and the like. Moreover, the term "solid" does not necessarily imply a complete absence of liquid or gaseous media. For example, solids can have various interstices, which may partially or fully fill with other gaseous and/or liquid media. Thus, the invention includes solid compositions that are suspended (i.e., remain at least partially, if not substantially, insoluble) in liquid media, such as syrups, elixirs, and the like.

The solid compositions of the invention may include UD1-FA in any suitable amounts. In some embodiments, UD1-FA is present in a therapeutically effective amount. As used herein, the term "therapeutically effective amount" refers to an amount of UD1-FA that elicits the biological or medicinal response in a tissue, system, or subject that is being sought by a researcher, veterinarian, medical doctor, patient or other clinician, which includes reduction or alleviation of the symptoms of the disease being treated.

As used herein, the term "subject" includes, for example, horses, cows, sheep, pigs, mice, dogs, cats, and primates such as chimpanzees, gorillas, rhesus monkeys, and humans. In some embodiments, the subject is a human. In some embodiments, the subject is a human in need of activation of glucokinase.

The actual amount of UD1-FA required, e.g., for treatment of any particular subject, will depend upon a variety of factors, including the following: the disorder being treated; its severity; the specific solid composition employed; the age, body weight, general health, gender, and diet of the subject; the mode of administration; the time of administration; the route of administration; the rate of excretion of the therapeutic agent; the duration of the treatment; any drugs used in combination or coincidental with the therapeutic agent; and other such factors well known to those skilled in the art. In various embodiments, for example, the solid composition may contain 1 mg or more, 5 mg or more, 10 mg or more, 20 mg or more, 40 mg or more, 50 mg or more, 100 mg or more, 200 mg or more, 300 mg or more, 400 mg or more, or 500 mg or more of UD1-FA in a given dosage form. In some embodiments, for example, the solid composition may contain less than 400 mg of UD1-FA, or less than 800 mg of UD1-FA in a given dosage form. In some further embodiments, the solid composition may contain about 100 mg, or about 150 mg, or about 200 mg, or about 250 mg, or about 300 mg, or about 350 mg, or about 400 mg, or about 450 mg, or about 500 mg of UD1-FA in a given dosage form.

UD1 (according to any of the above embodiments) may be useful for treating a variety of diseases or conditions where activation of glucokinase is beneficial. Thus, the solid compositions of the invention, when administered to a subject, e.g., in a therapeutically effective amount, are useful for treating type 1 diabetes, type 2 diabetes, metabolic syndrome, glucose intolerance, hyperglycaemia, dyslipidemia, hypertriglyceridemia, syndrome X, insulin resistance, impaired glucose tolerance (IGT), obesity, diabetic dyslipidemia, hyperlipidemia, arteriosclerosis, atherosclerosis, other cardiovascular diseases, hypertension, metabolic disorders where activation of GK is beneficial, or complications resulting from or associated with diabetes, including, but not limited to, neuropathy, retinopathy, nephropathy, and impaired wound healing.

### Water-Soluble Surfactant

In some embodiments of the invention, the solid composition comprises UD1 (according to any of the above embodiments), and further comprises a water-soluble surfactant. Surfactants are generally known in the art. Water-soluble surfactants are surfactants that dissolve in water when used at a desired concentration. Water-soluble surfactants, as a class, are well known in the art. The water-soluble surfactant may be selected from any suitable surfactant, including, but not limited to sulfuric acid alkyl ester salts, such as sodium lauryl sulfate; bile acid salts, such as sodium taurocholate and sodium glycocholate; propylene glycol fatty acid mono- or diesters, such as those sold under the trade name MIGLYOL® 840 (Sasol Olefins and Surfactants, Houston, Texas, USA); polyethylene glycol fatty acid esters, such as polyethylene glycol monooleate and polyethylene glycol monostearate; polysorbates, such as polyoxyethylene sorbitan fatty acid esters sold under the trade names TWEEN® 20, TWEEN 40®, and TWEEN® 80 (Spectrum Chemicals, Gardena, California, USA); polyoxyethylene-polyoxypropylene copolymer and block copolymer surfactants, such as poloxamer 188, poloxamer 235, poloxamer 404, and poloxamer 407 and those sold under the trade names PLURONIC® F87, PLURONIC® F127, PLURONIC® F68, PLURONIC® L44, PLURONIC® P123, and PLURONIC® P85 (BASF, Mt. Olive, New Jersey, USA); polyoxyethylene derivatives of natural oils and waxes, such as polyoxyethylene castor oil and polyoxyethylene hydrogenated castor oil, for example those sold under the trade names CREMOPHOR® RH40 and CREMOPHOR® EL (BASF, Mt. Olive, New Jersey, USA); polyoxyethylene derivatives of tocopherols or tocotrienols, such as vitamin E d-alpha tocopheryl polyethyleneglycol succinate (Vitamin E TPGS); and sorbitan fatty acid esters, such as sorbitan monooleate, sorbitan monostearate, sorbitan monopalmitate, sorbitan monolaurate, and sorbitan monocaprylate, sold under the trade names SPAN® 80, SPAN® 60, SPAN® 40, SPAN® 20, and SEFSOL® 418, respectively (Croda International PLC, Goole, UK). The selection and amount of the water soluble surfactant may be based, in part, upon its compatibility with the other ingredients in the solid composition, the amount of UD1-FA, the form of the UD1-FA (e.g., crystalline, etc.), and the consideration that the water-soluble surfactant is not generally deleterious to a human subject when the solid composition containing the surfactant is administered at typical dosing quantities. In some embodiments, the water-soluble surfactant is a polyoxyethylene sorbitan fatty acid ester, e.g., polysorbate 80. In some embodiments, the water-soluble surfactant is sodium lauryl sulfate. In some embodiments, the water-soluble surfactant is vitamin E d-alpha tocopheryl polyethyleneglycol succinate (vitamin E TPGS). In some embodiments, the water-soluble surfactant is a mixture of one or more of a polyoxyethylene sorbitan fatty acid ester, sodium lauryl sulfate, or vitamin E TPGS.

As used herein, the term "a mixture of" or "a mixture thereof" refers to any mixture of two or more materials and/or compositions that would be encompassed within the list that follows or precedes the phrase, respectively. The phrase does not refer to any particular type of mixture. Thus, the "mixture" is not necessarily an intimate mixture, a homogeneous mixture, etc. Furthermore, the "mixture" need not contain a representative of each element in the list. For example, if a composition comprises "A, B, C, or a mixture thereof," the term contemplates mixtures of A and B (with no C present), mixtures of B and C (with no A present), mixtures of A and C (with no B present), as well as mixtures of A, B, and C. As a further illustration, suppose that A, B, or C define generic categories (e.g., a polysorbate), where, for example, A¹ and A² are species or subgenuses encompassed by the genus A. In that instance, if a composition comprises "A, B, C, or a mixture thereof," the term also contemplates mixtures of A¹ and A² (where no B and no C are present in the mixture).

It was discovered that the presence of the water-soluble surfactant in the solid composition with a GK activator (e.g., UD1-FA) may surprisingly improve the resulting pharmacokinetic (PK) profile of the GK activator after the solid composition is administered to a subject. In some embodiments, the solid composition comprises between 0.1% and 10% by weight, or between 0.1% and 7% by weight, or between 0.3% and 5% by weight, or between 0.5% and 3.5% by weight, or between 1.0% and 3.0% by weight, or between 1.5% and 2.5% by weight, of water-soluble surfactant, based on the total weight of the solid composition. In some embodiments, the solid composition comprises about 0.5% by weight, or about 1% by weight, or about 1.5% by weight, or about 2% by weight, or about 2.5% by weight, or about 3% by weight, or about 3.5% by weight, or about 4% by weight, or about 5% by weight, of water-soluble surfactant, based on the total weight of the solid composition. In some further embodiments, the weight/weight ratio of UD1 to water-soluble surfactant in the solid composition ranges from 10:1 to 100:1, or 15:1 to 60:1, or from 18:1 to 50:1, or from 22:1 to 40:1, or from 27:1 to 35:1. In some embodiments, the weight/weight ratio of UD1 to water-soluble surfactant in the solid composition is about 20:1, or about 25:1, or about 30:1, or about 35:1, or about 40:1.

As noted below, in some embodiments, the solid composition comprises an evaporation residue. In some such embodiments, the evaporation residue comprises a water-soluble surfactant (according to any of the above embodiments).

### Pharmaceutically Acceptable Basic Excipient

In some embodiments of the invention, the solid composition comprises UD1 and a water-soluble surfactant (according to any of the above embodiments), and further comprises a pharmaceutically acceptable basic excipient. As used herein, the term "pharmaceutically acceptable basic excipient" refers to any metal salt of an acid which demonstrates basic properties, in either the Bronsted or Lewis sense, which includes those salts where all protons have been replaced with a mono or polyvalent metal ion and extends to those metal salts of acids which contain a proton but would lead to an aqueous solution having a pH greater than 7 when dissolved in water in appreciable amounts. Many such salts, particularly those of inorganic acids and many organic acids, may be water soluble. But water solubility is not a limiting factor in selecting a basic excipient. Metal salts of surfactants, whether water-soluble or water dispersible, are also within the scope of the basic excipients as defined herein. The pharmaceutically acceptable basic excipients of the invention are generally regarded as safe, at least in the dosage amounts used.

Pharmaceutically acceptable basic excipients include, but are not limited to, any of the salts of inorganic acids, short-chain mono-, di-, or tri-carboxylic acids, or salts of the various long-chain fatty acids or sulfonated fatty acids and alcohols and related surfactants. Selected salts should be inert in the sense that they themselves would not be expected or intended to demonstrate any deleterious or untoward pharmacological effects on the subject o which the dosage forms are administered.

Pharmaceutically acceptable basic excipients of inorganic acids include, for example: basic alkali metal salts of phosphoric acid, such as disodium phosphate, dipotassium phosphate, and calcium phosphate; basic alkali metal salts of orthophosphate, hypophosphate, and pyrophosphate, such as the di- and tri-sodium forms of orthophosphate, the di- and tri-potassium orthophosphates, magnesium orthophosphate, and magnesium pyrophosphate, sodium or potassium hypophosphate, sodium or potassium pyrophosphate, calcium hypophosphate and calcium orthophosphate, including the mono, di- and tri-calcium forms, calcium pyrophosphate, and mixed alkali metal salts of these various phosphates; alkali metal salts of nitric acids, such as sodium nitrate, potassium nitrate, calcium nitrate, and magnesium nitrate; alkali metal salts of sulfuric acid, such a sodium sulfate, potassium sulfate, magnesium sulfate, and calcium sulfate, and alkali metal salts of boric acid, such as sodium borate or potassium borate.

Pharmaceutically acceptable basic excipients further include basic alkali metal salts of various mono-, di-, or tri-carboxylic acids, for example, the alkali metal salts of carbonic acid, such as sodium bicarbonate, sodium carbonate, potassium carbonate, potassium bicarbonate, sodium potassium carbonate, magnesium carbonate or calcium carbonate may be used herein.

Pharmaceutically acceptable basic excipients further include alkali metal salts and alkaline earth metal salts of organic acids, such as formic acid, acetic acid, propionic acid, glycolic acid, lactic acid, pyruvic acid, oxalic acid, malonic acid, succinic acid, malic acid, maleic acid, fumaric acid, tartaric acid, benzoic acid, cinnammic acid, and mandelic acid.

As noted above, the invention provides solid compositions comprising UD1-FA and a water-soluble surfactant (according to any of the embodiments recited above) and at least one pharmaceutically acceptable basic excipient. In some such embodiments, the pharmaceutically acceptable basic excipient is selected from trisodium phosphate, potassium carbonate, sodium carbonate, sodium bicarbonate, potassium bicarbonate, or a mixture thereof. In other such embodiments, the pharmaceutically acceptable basic excipient is mixture of sodium carbonate and sodium bicarbonate. In some other such embodiments, the pharmaceutically acceptable basic excipient is sodium carbonate.

In various embodiments, the pharmaceutically acceptable basic excipient is present in the solid composition in an amount such that the relative amount of pharmaceutically acceptable basic excipient to UD1 (as a free acid and/or pharmaceutically acceptable salt) is suitable to allow for effective dissolution of the UD1 in the stomach and/or the upper part of the small intestine. The suitable ratio of UD1 to the total amount of pharmaceutically acceptable basic excipient(s) can depend on various factors, including but not limited to: the presence or absence of other excipients (and their relative quantities) in the solid composition; the dosage form in which the solid composition is packaged; the chemical identity of the pharmaceutically acceptable basic excipient or excipients (including the pK_{b} value(s)); the process for preparing the solid composition; and the total amount of UD1 present in the dosage form. In some embodiments, the weight/weight ratio of UD1 to total pharmaceutically acceptable basic excipient ranges from 1:3 to 25:1, or from 1:2 to 20:1, or from 1:1 to 17:1, or from 2:1 to 15:1. For example, in some embodiments, said ratio is about 1:2, or about 2:3, or about 1:1, or about 2:1, or about 5:1, or about 7:1, or about 10:1, or about 12:1, or about 15:1. In some embodiments, the weight/weight ratio of UD1 to total pharmaceutically acceptable basic excipient ranges from 1:1 to 3:1. In some other embodiments, the weight/weight ratio of UD1 to total pharmaceutically acceptable basic excipient ranges from 1:1 to 1:3. The amount of pharmaceutically acceptable basic excipient may also vary, in part, depending upon the particular basic excipient chosen.

### Binder

In some embodiments of the invention, the solid composition comprises UD1 and a water-soluble surfactant (according to any of the above embodiments), and further comprises a binder. Suitable binders include, but are not limited to, polyvinylpyrrolidone (PVP), hydroxypropylmethyl cellulose acetate succinate (HPMCAS), hydroxypropylmethyl cellulose phthalate (HPMCP), hydroxypropylmethyl cellulose (HPMC), poloxamers, hydroxypropyl methyl cellulose acetate, hydroxypropyl cellulose, and hydroxyethyl cellulose acetate, polyacrylates, methyl acrylatemethacrylic acid copolymers, ethyl acrylatemethacrylic acid copolymers, cellulose acetate phthalate, cellulose acetate trimellitate, carboxymethyl ethyl cellulose, hydroxyethyl cellulose (HEC), polyethylene oxide (polyox), polyethylene glycol, ethylcellulose, and mixtures thereof.

In some embodiments, the binder is hydroxypropylmethyl cellulose acetate succinate (HPMCAS) or polyvinylpyrrolidone (PVP) or hydroxypropylmethylcellulose (HPMC). In some embodiments, the binder is hydroxypropylmethyl cellulose acetate succinate (HPMCAS). In some embodiments, the binder is polyvinylpyrrolidone (PVP). In some embodiments, the binder is hydroxypropylmethylcellulose (HPMC).

In some embodiments of the invention, the amount of binder present in a solid composition is an amount such that the weight/weight ratio of UD1 to binder ranges from 25:1 to 400:1, or from 35:1 to 300:1, or from 50:1 to 250:1, or from 65:1 to 200:1, or from 75:1 to 150:1. In some embodiments, the weight/weight ratio of UD1 to binder is about 50:1, or about 75:1, or about 100:1, or about 125:1, or about 150:1, or about 200:1. The amount of binder in a solid composition of the invention may vary depending, in part, upon the specific features of the solid composition, including the amount of UD1.

### Evaporation Residue

In some embodiments of the invention, the solid compositions comprise an evaporation residue, which comprises UD1 (according to any of the embodiments recited above). In some such embodiments, the evaporation residue further comprises other excipients. In some such embodiments, the evaporation residue comprises UD1 and a water-soluble surfactant (according to any of the embodiments recited above). In some further such embodiments, the evaporation residue comprises UD1, a water-soluble surfactant, and one or both of a pharmaceutically acceptable basic excipient and/or a binder (each according to any of the embodiments recited above). In other embodiments, the evaporation residue comprises UD1, but does not contain any substantial amount of pharmaceutically acceptable basic excipient (e.g., less than 5% by weight, or less than 3% by weight, or less than 1% by weight, or less than 0.5% by weight, of the total weight of the evaporation residue).

As used herein, the term "evaporation residue" refers to the solids remaining after the substantial removal of solvent from a solution and/or suspension comprising UD1, alone or in combination with other components. For example, the evaporation residue contains less than 1 % by weight, or less than 0.5 % by weight, or less than 0.2 % by weight of solvent, based on the total weight of the evaporation residue. In some embodiments, removal of the solvent from the solution or suspension comprises spray drying the solution or suspension to form a powder. In other embodiments, the solution is removed by evaporation, for example by using a rotovap or a flat-bed dryer to form an evaporation residue.

### Additional Ingredients

In some embodiments of the invention, the solid composition further comprises at least one additional pharmaceutical ingredient. As used herein, the term "additional pharmaceutical ingredient" refers to a component or excipient other than powdered pharmaceutically acceptable carriers, so long as the material is not generally deleterious to a human subject when the solid composition is administered at dosing quantities. Non-limiting examples of additional ingredients include:
a) glidants and lubricants, such as colloidal silica, talc, magnesium stearate, calcium stearate, stearic acid, solid polyethylene glycol, sodium oleate, sodium stearate, sodium benzoate, sodium acetate, sodium chloride, sodium stearyl furamate, and sodium lauryl sulfate;
b) disintegrating and solubilizing agents, such as agar-agar, calcium carbonate, sodium carbonate, croscarmellose sodium, starches, pregelatinized starches, sodium starch glycolate, crospovidone, methyl cellulose, agar, bentonite, xanthan gum, alginic acid, and certain silicates;
c) solution retarding agents, such as polymers, for example biodegradable polymers such as polylactic acid, polyepsilon caprolactone, polyhydroxy butyric acid, polyorthoesters, polyacetals, polydihydropyrans, polycyanoacrylates, and cross-linked or amphipathic block copolymers of hydrogelsparaffin, and wax, for example, paraffin;
d) resorption accelerating agents, such as quaternary ammonium compounds;
e) absorption agents, such as quaternary ammonium compounds, bentonite, kaolin, or dicalcium phosphate;
f) fillers, such as anhydrous lactose, microcrystalline cellulose, mannitol, calcium phosphate, pregelatinized starch, and sucrose.

It is within the ability of one of skill in the art to select the at least one additional pharmaceutical ingredient and the amount of said additional ingredient. The selection and amount of the at least one additional pharmaceutical ingredient is based, in part, upon its compatibility with the other ingredients in the formulation, the amount of UD1, and consideration that it is not generally deleterious to a human subject when the solid composition is administered at dosing quantities.

### Methods of Making the Solid Composition

The solid compositions of the invention can be made by various means known in the pharmaceutical formulation arts. Suitable methods include, but are not limited to the following: wet granulation methods, including standard wet granulation techniques, and specialized wet granulation techniques, such as high-shear mixture granulation, fluid-bed granulation, extrusion, and spheronization, spray granulation (e.g., spray-drying granulation), and the like; dry granulation techniques, including standard dry granulation and specialized dry granulation techniques, such as slugging, roller compaction, and the like; steam granulation techniques; melt granulation techniques, such as thermoplastic melt granulation; moisture-activated dry granulation techniques (MADG); moist granulation techniques (MGT); thermal adhesion granulation processes (TAGP); foam granulation techniques; and the like. In some embodiments of the invention, a wet granulation technique is used to make a solid composition comprising UD1 (according to any of the embodiments recited above). In some embodiments, a fluid-bed wet granulation technique is used to make a solid composition comprising UD1 (according to any of the embodiments recited above). In some embodiments, a spray granulation technique is used to make a solid composition comprising UD1 (according to any of the embodiments recited above).

The aforementioned granulation techniques may generate a solid composition that comprises granules that contain UD1 (according to any of the embodiments recited above). The particle size and the distribution of particle sizes of the granules can be adjusted according to known techniques to achieve release profiles, dissolution, and the like. In some such embodiments, at least 80%, or at least 85%, or at least 90%, or at least 95% (by weight) of said granules have a particle size that is between 1 µm and 1 mm. Further, in some such embodiments, at least 80%, or at least 85%, or at least 90%, or at least 95% (by weight) of said granules have a particle size that is between 1 µm and 500 µm.

### Wet Granulation

As noted above, in some embodiments, a wet granulation technique is used to make a solid composition comprising UD1. In general, wet granulation involves the use of a liquid binder solution, which is mixed with a powder to cause the powder to agglomerate lightly, thereby forming granules. Following granule formation, the granules are typically dried, sized (using, e.g., mesh screens). In some instances, the granules can be milled, so as to achieve a desired size. Both low-shear and high-shear mixing equipment are suitable.

Wet granulation typically requires the use of a binder solution. Suitable binders are well known in the art, and include, but are not limited to aqueous solutions of corn starch, various natural gums, such as acacia, various cellulose derivatives, such as methyl cellulose and hypromellose, gelatin, povidone, and the like. Binder solutions can also contain surfactants, such as those described above. The amount of binder solution will vary depending on various factors known to those of skill in the art, including, but not limited to, the composition of the dry ingredients, the composition and concentration of the binder solution, the mixing speed, etc.

Wet granulation can occur in a single phase or in multiple phases. In a typical single-phase process, all dry ingredients are mixed with the binder solution prior to drying (e.g., in a fluid-bed dryer). In this way, the resulting granules have a relatively homogeneous composition throughout. But in a multiple-phase process, such as a two-phase process, there is a first mixing step followed by a drying step. The resulting granules are then subjected to another mixing step (with at least one other dry ingredient), which is then followed by a second drying step. Such a two-phase process can lead to granules that do not necessarily have a homogeneous composition throughout (as the first mixing step and the second mixing step can contain different solid ingredients and/or different amounts of solid ingredients).

### Spray-Dry Granulation

As noted above, in some embodiments, a spray-dry granulation technique is used to make a solid composition comprising UD1. In general, spray-dry granulation involves spraying a liquid solution onto a solid powder, which typically causes powder particles to agglomerate lightly. In most instances, the drying occurs during the agglomeration process, although it can be desirable, in some instances, to dry the resulting granules to drive out residual moisture (e.g., in a fluid bed). Following granule formation, the granules can be sized (using, e.g., mesh screens). In some instances, the granules are milled, so as to achieve a desired size.

Spray-dry granulation techniques may employ a binder solution or suspension, which is sprayed onto solid particles. The binder solution or suspension contains a binder material and other materials dissolved or suspended in a solvent. Once the solvent evaporates, the remaining components in the binder solution or suspension form an evaporation residue, as described above. Acceptable solvents include, but are not limited to, water or other polar solvents such as alcohols, for example ethanol and isopropanol, ketones, for example acetone, and mixtures thereof. In various embodiments, the solvent is selected from water, ethanol, acetone or mixtures thereof. In some embodiments, the solvent is water. In other embodiments, the solvent is a less polar solvent, such as THF.

The binder solution or suspension may comprise a binder. In some embodiments, the binder solution or suspension also comprises UD1. In some such embodiments, the binder solution or suspension further comprises other excipients, such as a pharmaceutically acceptable basic excipient. In other embodiments, the binder solution or suspension comprises UD1, but does not contain any substantial amount of pharmaceutically acceptable basic excipient (e.g., less than 5% by weight, or less than 3% by weight, or less than 1% by weight, or less than 0.5% by weight, of the total weight of the evaporation residue). In some further embodiments, the evaporation residue of any of the aforementioned embodiments may or may not further comprise a binder.

As noted above, binders include, but are not limited to, polyvinylpyrrolidone (PVP), hydroxypropylmethyl cellulose acetate succinate (HPMCAS), hydroxypropylmethyl cellulose phthalate (HPMCP), hydroxypropylmethyl cellulose (HPMC), poloxamers, hydroxypropyl methyl cellulose acetate, hydroxypropyl cellulose, and hydroxyethyl cellulose acetate, polyacrylates, methyl acrylatemethacrylic acid copolymers, ethyl acrylatemethacrylic acid copolymers, cellulose acetate phthalate, cellulose acetate trimellitate, carboxymethyl ethyl cellulose, hydroxyethyl cellulose (HEC), polyethylene oxide (polyox), polyethylene glycol, ethylcellulose, and mixtures thereof.

In some embodiments, the binder is hydroxypropylmethyl cellulose acetate succinate (HPMCAS) or polyvinylpyrrolidone (PVP) or hydroxypropylmethylcellulose (HPMC). In some embodiments, the binder is HPMCAS. In other embodiments, the binder is PVP. In other embodiments, the binder is HPMC.

In some embodiments, the spray-dry granulation process comprises spraying a solution or suspension onto a solid pharmaceutically acceptable carrier. As used herein and as known in the art, the term "pharmaceutically acceptable carrier" refers to pharmaceutically acceptable basic excipients, as described herein, pharmaceutically acceptable inert carriers, and/or mixtures thereof. As used herein and as known in the art, the term "pharmaceutically acceptable inert carriers" refers to those inorganic and organic carriers that are physiologically harmless and are not basic excipients. In addition to the pharmaceutically acceptable basic excipients listed above, solid pharmaceutically acceptable carriers include, but are not limited to edible carbohydrates, for example, starches, lactose, sucrose, glucose, and mannitol, silicic acid, calcium carbonate, calcium phosphate, sodium phosphate, crospovidone, and kaolin.

In some embodiments, the solid composition is formed by mixing a pharmaceutically acceptable basic excipient with a powdered pharmaceutically acceptable carrier onto which a solution or suspension containing UD1 and, optionally, a binder is sprayed. The evaporation residue is formed on and mixed with the powdered pharmaceutically acceptable carrier, which may be premixed with the pharmaceutically acceptable basic excipient or mixed after the spry drying step.

In yet other embodiments, a pharmaceutically acceptable basic excipient is mixed with an evaporation residue containing UD1 and, optionally, a binder.

### Dosage Forms

The invention further provides solid compositions in forms for oral administration, for example, as discrete units, such as capsules or tablets. Preparation of the solid compositions in forms intended for oral administration is within the ability of one skilled in the art, including the selection of pharmaceutically acceptable additional ingredients from the groups listed above in order to provide pharmaceutically elegant and palatable preparations. For example, the solid compositions of the invention may be prepared by methods known in the pharmaceutical formulation art, for example, see Remington's Pharmaceutical Sciences, 18th ed., (Mack Publishing Company, Easton, Pa., 1990).

In various embodiments, capsules may be prepared by, for example, preparing a powder mixture comprising UD1 and a water-soluble surfactant (according to any of the above embodiments) and encapsulating the powder with gelatin or some other appropriate shell material. Additional ingredients, such as those set forth above and including glidants and lubricants and disintegrating and solubilizing agents, may be added to the powder before the encapsulation.

In various other embodiments, tablets may be prepared by, for example, preparing a powder mixture, such as that described above in various embodiments, and pressing the mixture into tablets. Additional ingredients, such as those set forth above and including glidants and lubricants, disintegrating and solubilizing agents, binders, solution retardants, and absorption agents, may be added to the powder before pressing into tablets. The powder mixture may be wet-granulated with a binder such as syrup, starch paste, acadia mucilage or solutions of cellulosic or polymeric materials, and forcing through a screen. Or, in other embodiments, the powder mixture may be run through the tablet machine, producing slugs broken into granules. Then granules may be lubricated and then compressed into tablets. In a further embodiment, the powder mixture may be compressed directly into tablets without granulation or slugging.

In some embodiments of the invention, the tablets are multipart or multilayer tablets. For example, UD1 mixed with a water-soluble surfactant, and at least one additional ingredient, are compressed to form one part or one layer of a multipart or multilayer tablet. At least one pharmaceutically acceptable basic excipient is compressed to form another part or another layer of a multipart or multilayer tablet. In at least one embodiment, the UD1 part or layer and the basic excipient part or layer are combined to form a multipart or multilayer tablet. In a further embodiment, the UD1 part or layer and the basic excipient part or layer are separated by an additional part or layer comprising additional ingredients, e.g., ingredients that will react with UD1 or metformin.

The tablets of the invention may be either uncoated or coated. In various embodiments, tablets are coated with a clear or opaque protective coating, which may for example, comprise a sealing coat of shellac, a coating of sugar or polymeric material, and/or a polish coating of wax. In various embodiments, tablets are coated to delay disintegration and absorption in the gastrointestinal tract and thereby provide a sustained action over a longer period. Such coatings may comprise glyceryl monostearate or glyceryl distearate. Additionally, dyestuffs can be added to these coatings to distinguish different unit dosages.

The solid compositions of the invention may exhibit improved bioavailability of UD1 upon administration to a subject relative to solid compositions that do not include UD1 and a water-soluble surfactant.

As used herein, the term "improved bioavailability" means that the bioavailability of UD1 delivered in the solid composition of the invention is increased and may be approximately at least 1.3 times, or 1.5 times, or double, relative to the bioavailability of conventional compositions, for example at least three times, at least five times, or at least ten times that of conventional compositions. It is within the ability of one of skill in the art to determine the bioavailability of a compound or composition using methods generally accepted in the art. For example, the maximum concentration (Cₘₐₓ) of UD1 in plasma or the overall amount of UD1 in plasma after a dosage, e.g., area-under-the-curve (AUC), may be used for the comparison. These pharmacokinetic measurements may be determined by conventional techniques. For example, in various embodiments, the concentration of UD1 in plasma may be determined by a LC-MS/MS assay following a protein precipitation step with acetonitrile. In additional embodiments, pharmacokinetic analysis may be performed using the WinNonlin™ software program, which is available from Pharsight, Inc. of Mountain View, California, USA. The area under the plasma concentration-time curve (AUC₀₋ₜ) may be calculated from the first time point (0 min) up to the last time point with measurable drug concentration. The AUC_{0-inf} may be calculated as the sum of AUC₀₋ₜ and Cpred/λz, where Cpred was the predicted concentration at the time of the last quantifiable concentration.

In some embodiments, improvements in bioavailability may be based, in part, upon the selection of and amount of at least one water-soluble surfactant and optional at least one of a pharmaceutically acceptable basic excipient or a binder.

### Methods of Treatment

The invention further relates to methods of treating type 2 diabetes or high blood glucose levels using any one of the solid compositions of the invention. For example, in at least one aspect, the invention relates to methods of treating type 2 diabetes or high blood glucose levels, where the method comprises administering to a subject (e.g., a human) a solid composition comprising a therapeutically effective amount of UD1.

The invention further relates to methods of treating type 1 diabetes or high blood glucose levels using any one of the solid compositions of the invention. For example, in at least one aspect, the invention relates to methods of treating type 1 diabetes or high blood glucose levels, where the method comprises administering to a subject (e.g., a human) a solid composition comprising a therapeutically effective amount of UD1.

The invention also relates to a method of lowering blood glucose concentration in a subject comprising administering to a subject (e.g., a human) any one of the solid compositions of the invention. For example, the invention relates to a method of lowering blood glucose concentration in a subject comprising administering to a subject a solid composition comprising a therapeutically effective amount of UD1. In a further embodiment, the method lowers fasting blood glucose concentration in a subject. In another embodiment, the method lowers postprandial blood glucose concentration in a subject. In another embodiment, the subject is suffering from type 2 diabetes.

The invention also relates to a method of activating glucokinase in a subject comprising administering to a subject (e.g., a human) any one of the solid compositions of the invention. For example, the invention relates to a method of activating glucokinase in a subject comprising administering to a subject a solid composition comprising a therapeutically effective amount of UD1. In various embodiments, the subject is suffering from type 2 diabetes.

The invention further relates to a method of activating hepatic glucokinase in a subject comprising administering to a subject (e.g., a human) any one of the solid compositions of the invention. For example, the invention relates to a method of activating hepatic glucokinase in a subject comprising administering to a subject a solid composition comprising a therapeutically effective amount of UD1. In various embodiments, the subject is suffering from type 2 diabetes.

The invention also relates to a method of increasing hepatic glucose use in a subject comprising administering to a subject (e.g., a human) any one of the solid compositions of the invention. For example, the invention relates to a method of increasing hepatic glucose use in a subject comprising administering to a subject a solid composition comprising a therapeutically effective amount of UD1. In various embodiments, the subject is suffering from type 2 diabetes.

The invention also relates to a method of treating a disease, disorder, or condition comprising administering to a subject (e.g., a human) any one of the solid compositions of the invention, where the disease, disorder, or condition is selected from metabolic syndrome, glucose intolerance, hyperglycaemia, dyslipidemia, hypertriglyceridemia, syndrome X, insulin resistance, impaired glucose tolerance (IGT), obesity, diabetic dyslipidemia, hyperlipidemia, arteriosclerosis, atherosclerosis, other cardiovascular diseases, hypertension, metabolic disorders where activation of GK is beneficial, or complications resulting from or associated with diabetes, including, but not limited to, neuropathy, retinopathy, nephropathy, and impaired wound healing.

The solid compositions administered in these methods of the invention are the same in the various embodiments, and have the same preferred embodiments, as those discussed above. Thus, in an embodiment of any of the above methods, a solid composition may be administered wherein the solid composition comprises UD1 and a binder, and optionally at least one of a pharmaceutically acceptable basic excipient or a water-soluble surfactant.

In another embodiment of any of the methods of treatment above, a solid composition may be administered wherein the solid composition comprises at least one pharmaceutically acceptable basic excipient and an evaporation residue comprising UD1. In a further embodiment, the evaporation residue may further comprise at least one binder.

### EXAMPLES

The following examples are provided only as illustrations of the invention, and are not intended to limit the scope of the patent claims in any way. The claims describe the literal scope of the invention and provide the elements against which any equivalents are to be compared.

The following commercially available materials were used in the examples below:
HPMCAS polymeric binders (AQOAT, MG and LG type), available from Shinetsu Chemical Industries Co., Ltd., Tokyo, Japan;
Avicel PH101, microcrystalline cellulose, available from FMC Biopolymer, Newark DE, USA;
Cabosil, fumed silica, available from Cabot of Tuscola, IL, USA;
Plasdone K29-32, polyvinylpyrrolidone, available from Spectrum Chemicals of Gardena, CA, USA;
Pluronic F127, a poloxamer surfactant, available from BASF of Mt. Olive, NJ, USA; and
Polysorbate 80 (TWEEN 80) surfactant, available from Spectrum Chemicals of Gardena, CA, USA.

### Example A - PXRD of Unmicronized UD1

A containing crystalline {2-[3-cyclohexyl-3-(*trans*-4-propoxy-cyclohexyl)-ureido]-thiazol-5-ylsulfanyl}-acetic acid was analyzed by powder x-ray diffraction using Cu-Kα radiation as the incident radiation. Prior to analysis, the sample was not micronized. The x-ray diffractogram was recorded and the data were analyzed using standard data analysis software. Table 1 recites the recorded diffraction angles, the corresponding d-spacings in the sample, and the relative intensities of the peaks in the diffractogram.

**Table 1**

| Angle (2θ°) | d value (Å) | Rel Intensity | Intensity % |
|---|---|---|---|
| 2.240 | 39.409 | 10.7 | 3.6 |
| 2.557 | 34.520 | 14.4 | 4.9 |
| 2.812 | 31.396 | 20.4 | 7.0 |
| 3.081 | 28.650 | 9.85 | 3.4 |
| 3.381 | 26.109 | 6.24 | 2.1 |
| 3.869 | 22.818 | 9.47 | 3.2 |
| 4.260 | 20.724 | 3.38 | 1.2 |
| 4.556 | 19.381 | 9.11 | 3.1 |
| 4.924 | 17.932 | 9.33 | 3.2 |
| 6.000 | 14.719 | 13.9 | 4.7 |
| 8.576 | 10.303 | 241 | 81.9 |
| 9.267 | 9.5359 | 147 | 50.1 |
| 11.248 | 7.8604 | 18.2 | 6.2 |
| 12.059 | 7.3334 | 210 | 71.6 |
| 12.283 | 7.1999 | 183 | 62.4 |
| 12.953 | 6.8290 | 106 | 36.2 |
| 14.420 | 6.1377 | 153 | 52.1 |
| 15.704 | 5.6385 | 20.2 | 6.9 |
| 16.827 | 5.2647 | 153 | 52.1 |
| 17.390 | 5.0953 | 165 | 56.3 |
| 18.645 | 4.7551 | 160 | 54.3 |
| 19.117 | 4.6388 | 184 | 62.8 |
| 19.481 | 4.5530 | 60.0 | 20.4 |
| 20.111 | 4.4118 | 293 | 100 |
| 20.754 | 4.2764 | 122 | 41.6 |
| 21.347 | 4.1591 | 73.0 | 24.9 |
| 21.726 | 4.0872 | 174 | 59.4 |
| 22.159 | 4.0085 | 12.0 | 4.1 |
| 22.662 | 3.9206 | 49.8 | 17.0 |
| 22.999 | 3.8639 | 40.9 | 13.9 |
| 23.400 | 3.7985 | 27.3 | 9.3 |
| 23.677 | 3.7547 | 55.3 | 18.8 |
| 23.931 | 3.7154 | 57.4 | 19.5 |
| 24.312 | 3.6581 | 36.8 | 12.5 |
| 24.846 | 3.5806 | 12.8 | 4.4 |
| 25.248 | 3.5245 | 5.44 | 1.9 |
| 25.352 | 3.5103 | 4.47 | 1.5 |
| 25.907 | 3.4364 | 32.2 | 11.0 |
| 27.170 | 3.2794 | 68.6 | 23.4 |
| 27.520 | 3.2385 | 37.9 | 12.9 |
| 28.213 | 3.1606 | 24.4 | 8.3 |
| 29.117 | 3.0644 | 31.8 | 10.8 |
| 34.789 | 2.5767 | 15.8 | 5.4 |
| 38.069 | 2.3619 | 8.85 | 3.0 |
| 40.734 | 2.2133 | 16.7 | 5.7 |
| 44.637 | 2.0284 | 18.9 | 6.4 |

### Example B - PXRD of Micronized UD1

A sample containing crystalline {2-[3-cyclohexyl-3-(*trans*-4-propoxy-cyclohexyl)-ureido]-thiazol-5-ylsulfanyl}-acetic acid was analyzed by powder x-ray diffraction using Cu-Kα radiation as the incident radiation. Prior to analysis, the sample was micronized using an air jet. Air jet micronization typically produces particles that range in size from about 1 to about 100 µm. The x-ray diffractogram for the micronized sample was recorded and the data were analyzed using standard data analysis software. Table 2 recites the recorded diffraction angles, the corresponding d-spacings in the sample, and the relative intensities of the peaks in the diffractogram.

**Table 2**

| Angle (2θ°) | d value (Å) | Rel Intensity | Intensity % |
|---|---|---|---|
| 2.320 | 38.050 | 14.2 | 7.1 |
| 2.400 | 36.782 | 7.47 | 3.7 |
| 2.500 | 35.311 | 17.7 | 8.9 |
| 2.620 | 33.694 | 18.9 | 9.5 |
| 2.908 | 30.361 | 19.6 | 9.8 |
| 3.151 | 28.020 | 7.21 | 3.6 |
| 3.400 | 25.966 | 10.4 | 5.2 |
| 3.900 | 22.638 | 14.3 | 7.2 |
| 4.592 | 19.226 | 13.2 | 6.6 |
| 4.858 | 18.176 | 9.23 | 4.6 |
| 5.340 | 16.536 | 10.7 | 5.4 |
| 7.029 | 12.565 | 13.5 | 6.8 |
| 7.578 | 11.657 | 7.2 | 3.6 |
| 8.581 | 10.296 | 180 | 90.2 |
| 9.255 | 9.5483 | 111 | 55.7 |
| 11.197 | 7.8956 | 12 | 6.0 |
| 12.071 | 7.3259 | 162 | 81.1 |
| 12.296 | 7.1926 | 145 | 73 |
| 12.952 | 6.8299 | 98.9 | 49.6 |
| 14.399 | 6.1463 | 88.7 | 44.5 |
| 14.920 | 6.1463 | 5.22 | 2.6 |
| 16.853 | 5.2564 | 135 | 67.7 |
| 17.382 | 5.0978 | 128 | 64.2 |
| 18.645 | 4.7553 | 95.2 | 47.8 |
| 19.127 | 4.6364 | 134 | 67.5 |
| 19.502 | 4.5482 | 52.5 | 26.4 |
| 20.100 | 4.4142 | 199 | 100 |
| 20.775 | 4.2722 | 78.6 | 39.5 |
| 21.400 | 4.1489 | 56.0 | 28.1 |
| 21.727 | 4.0870 | 137 | 68.6 |
| 22.147 | 4.0106 | 8.78 | 4.4 |
| 22.674 | 3.9185 | 46.7 | 23.4 |
| 23.040 | 3.8571 | 30.2 | 15.1 |
| 23.795 | 3.7364 | 45.1 | 22.7 |
| 24.319 | 3.6570 | 22.1 | 11.1 |
| 24.809 | 3.5859 | 13.8 | 6.9 |
| 25.087 | 3.5468 | 6.74 | 3.4 |
| 25.760 | 3.4557 | 21.4 | 10.8 |
| 25.886 | 3.4392 | 20.8 | 10.4 |
| 26.566 | 3.3526 | 7.4 | 4.0 |
| 27.224 | 3.2731 | 43.8 | 22 |
| 27.520 | 3.2385 | 37.9 | 12.9 |
| 27.577 | 3.2319 | 30.2 | 15.2 |
| 29.342 | 3.0415 | 22.3 | 11.2 |
| 31.328 | 2.8530 | 13.1 | 6.6 |
| 32.860 | 2.7234 | 25.7 | 12.9 |
| 34.695 | 2.5834 | 15.3 | 7.7 |
| 36.845 | 2.4375 | 14.1 | 7.1 |
| 37.869 | 2.3739 | 15.5 | 7.8 |
| 43.839 | 2.0635 | 11.0 | 5.5 |

### Example 1

0.51 g of HPMC (METHOCEL E3 LV, USP, Dow Chemical Co., Midland, MI, USA) and 0.41 g of sodium lauryl sulfate were dissolved in 101.1 g of water. 18.0 g of UD1-FA was added to this solution to form a suspension. This suspension was milled for 1.5 hours using a bead mill (Dyno-Mill, Glenn Mills Inc.). 1.0 g of TWEEN 80 was dissolved in 67.0 g of the prepared nanosuspension. The resulting nanosuspension was then spray dried onto a mixture of 6.0 g of AVICEL PH101, 6.0 g of lactose, 2.8 g of crospovidone, and 2.0 g of pregelatinized starch using a fluidized bed granulation (Vector Laboratory Micro Fluid Bed) equipment to obtain mixture of fine powder and small granules. 16.6 g of this powder was thoroughly blended with 3.6 g of AVICEL PH101, 1.8 g of pregelatinized starch, 1.8 g of crospovidone, and 0.11 g of magnesium stearate. This final blend was compressed into tablets using SC-2 single station tablet press from Key International; each tablet had hardness of 8-12 Kp. Each tablet weighed 451 mg and contained 100 mg of UD1-FA.

### Example 2

12.14 g of UD1-FA, 1.08 g of TWEEN 80, and 0.08 g of HPMCAS were dissolved in 485 mL of THF. The solution was spray dried onto a mixture of 7.20 g of AVICEL PH101, 7.20 g of lactose DT, and 3.0 g of crospovidone using fluidized bed granulation (Vector Laboratory Micro Fluid Bed) equipment. The granules were passed through a #60 mesh screen to obtain a mixture of fine powder and small granules. 2.55 g of this powder was thoroughly blended with 0.62 g of AVICEL PH101, 0.33 g of crospovidone, 0.33 g of corn starch, 0.04 g of CAB-O-SIL, 0.10 g of sodium lauryl sulfate, and 0.02 g of magnesium stearate. The resulting mixture was compressed into tablets using SC-2 single station tablet press from Key International; each tablet had hardness of 8-12 Kp. Each tablet weighed 400 mg and contained 100 mg of UD1-FA.

### Example 3

15.0 g of UD1-FA, 45.0 g of HPMCAS, MG grade, and 0.30 g of TWEEN 80 were dissolved in 600 mL of THF. The solution was spray dried in a spray dryer (Niro spray drier) and dried to obtain a fine powder. 2.42 g of the powder was thoroughly blended with 0.24 g of AVICEL PH101, 0.24 g of crospovidone, 0.24 g of pregelatinized starch, 0.24 g of corn starch, and 0.01 g of magnesium stearate. The powder was compressed in a tablet press, milled and passed through a #30 mesh screen. The powder was then blended with 0.19 g of AVICEL PH101, 0.11 g of pregelatinized starch, 0.21 g of corn starch, 0.21 g of crospovidone, 0.04 g of CAB-O-SIL, 0.10 g of sodium lauryl sulfate, and 0.01 g of magnesium stearate. The resulting mixture was compressed into tablets using SC-2 single station tablet press from Key International; each tablet had hardness of 8-12 Kp. Each tablet weighed 710 mg and contained 100 mg of UD1-FA.

### Example 4

57.6 g of TWEEN 80 and 14.4 g of HPMC E3 LV were dissolved in 1100 mL of water. 1600.0 g of UD1-FA, 280.0 g of AVICEL PH101, 299.2 g of lactose monohydrate, and 184.0 g of AC-DI-SOL were transferred to a high shear granulator. The powder was blended for 2 minutes at 250 rpm with the chopper off. The HPMC/TWEEN 80 solution was then pumped into the granulator while mixing for 1-2 minutes with an impeller speed of 250 rpm and chopper speed of 1000 rpm. Additional water was added to complete the granulation. The wet granules were transferred to a Vector FL-Multi-3 Fluid bed drier and dried the granules to LOD of <3.0% using inlet temperature of 50-60 °C. The dried granules were passed through a #30 mesh screen. 2189.4 g of the wet granulation were thoroughly blended with 128.02 g of AVICEL PH101, 129.46 g of AC-DI-SOL, 129.46 g of pregelatinized starch (Starch 1500), and 12.95 g of magnesium stearate. The resulting mixture was then filled in Swedish orange opaque capsules using encapsulator equipment. Each capsule weighed 360 mg and contained 200 mg of UD1-FA.

### Example 5

12.14 g of UD1-FA, 1.44 g of TWEEN 80, 1.44 g of Vitamin E TPGS, and 0.35 g of HPMCAS were dissolved in 485 mL of THF. The solution was spray dried onto a mixture of 7.20 g of AVICEL PH101, 7.20 g of lactose DT, and 3.0 g of crospovidone using fluidized bed granulation (Vector Laboratory Micro Fluid Bed) equipment. The granules were passed through a #60 mesh screen to obtain mixture of fine powder and small granules. 2.73 g of this powder was thoroughly blended with 0.59 g of AVICEL PH101, 0.33 g of crospovidone, 0.33 g of corn starch, 0.04 g of CAB-O-SIL, 0.10 g of sodium lauryl sulfate, and 0.02 g of magnesium stearate. The resulting mixture was compressed into tablets using SC-2 single station tablet press from Key International; each tablet had hardness of 8-12 Kp. Each tablet weighed 415 mg and contained 100 mg of UD1-FA.

### Example 6

12.14 g of UD1-FA, 1.08 g of TWEEN 80, and 0.08 g of HPMCAS were dissolved in 485 mL of THF. The solution was spray dried onto a mixture of 7.20 g of AVICEL PH101, 7.20 g of lactose DT, and 3.0 g of crospovidone using fluidized bed granulation (Vector Laboratory Micro Fluid Bed) equipment. The granules were passed through a #60 mesh screen to obtain a mixture of fine powder and small granules. 2.55 g of this powder was thoroughly blended with 0.23 g of AVICEL PH101, 0.16 g of crospovidone, 0.38 g of corn starch, 0.05 g of CAB-O-SIL, 0.14 g of sodium lauryl sulfate, 1.50 g of anhydrous sodium carbonate, 0.50 g of anhydrous sodium bicarbonate, and 0.03 g of magnesium stearate. The resulting mixture was compressed into tablets using SC-2 single station tablet press from Key International; each tablet had hardness of 8-12 Kp. Each tablet weighed 555 mg and contained 100 mg of UD1-FA.

### Example 7

0.51 g of HPMC E3 and 0.41 g of sodium lauryl sulfate were dissolved in 101.1 g of water. 18.0 g of UD1-FA was added to this solution to form a suspension. This suspension was milled for 1.5 hours using a bead mill (Dyno-Mill, Glenn Mills Inc.). 1.0 g of TWEEN 80 was dissolved in 67.0 g of the prepared nanosuspension. The resulting nanosuspension was then spray dried onto a mixture of 6.0 g of AVICEL PH101, 6.0 g of lactose, 2.8 g of crospovidone, and 2.0 g of pregelatinized starch using a fluidized bed granulation (Vector Laboratory Micro Fluid Bed) equipment to obtain mixture of fine powder and small granules. 1.71 g of this powder was thoroughly blended with 0.63 g of AVICEL PH101, 0.31 g of pregelatinized starch, 0.31 g of crospovidone, 0.90 g of anhydrous sodium carbonate, 0.30 g of anhydrous sodium bicarbonate, and 0.02 g of magnesium stearate. This final blend was compressed into tablets using SC-2 single station tablet press from Key International; each tablet had hardness of 8-12 Kp. Each tablet weighed 697 mg and contained 100 mg of UD1-FA.

### Example 8

12.14 g of UD1-FA, 1.44 g of TWEEN 80, 1.44 g of Vitamin E TPGS, and 0.35 g of HPMCAS were dissolved in 485 mL of THF. The solution was spray dried onto a mixture of 7.20 g of AVICEL PH101, 7.20 g of lactose DT, and 3.0 g of crospovidone using fluidized bed granulation (Vector Laboratory Micro Fluid Bed) equipment. The granules were passed through a #60 mesh screen to obtain mixture of fine powder and small granules. 2.73 g of this powder was thoroughly blended with 0.26 g of AVICEL PH101, 0.16 g of crospovidone, 0.37 g of corn starch, 0.06 g of CAB-O-SIL, 0.14 g of sodium lauryl sulfate, 1.50 g of anhydrous sodium carbonate, 0.50 g of anhydrous sodium bicarbonate, and 0.03 g of magnesium stearate. The resulting mixture was compressed into tablets using SC-2 single station tablet press from Key International; each tablet had hardness of 8-12 Kp. Each tablet weighed 575 mg and contained 100 mg of UD1-FA.

### Example 9

0.36 g of TWEEN 80 and 0.09 g of HPMC E3 were dissolved in 8 mL of water. 10.12 g of UD1-FA, 1.75 g of AVICEL PH101, 1.75 g of lactose SD, and 1.15 g of AC-DI-SOL were transferred to the blender (Variac). They were mixed at low speed for 1 minute (Variac at 50% setting) and any adhering powder was scrapped from the sides of the blender. The HPMC/TWEEN 80 solution was then added to the blender while mixing at low speed (60-70% setting) in 2 minutes. After adding the solution completely, it was mixed for another 1 minute. Additional water was added to it to complete the granulation (target 15 mL) and mixed for another 1 minute. The wet granules were then transferred to fluid bed drier and dried the granules to LOD of <3.0% using inlet temperature of 70 °C. The dried granules were passed through a #30 mesh screen. 12.17 g of wet granulation was thoroughly blended with 2.55 g of AVICEL PH101, 2.56 g of pregelatinized starch (Starch 1500 LM), 2.56g of AC-DI-SOL, 1.20 g of corn starch, 0.24 g of CAB-O-SIL M5P, 1.20 g of sodium lauryl sulfate, 12.0 g of anhydrous sodium carbonate, 4.0 g of anhydrous sodium bicarbonate, and 0.23 g of magnesium stearate. The resulting mixture was compressed into tablets using SC-2 single station tablet press from Key International; each tablet had hardness of 8-12 Kp. Each tablet weighed 484 mg and contained 100 mg of UD1-FA.

### Example 10

12.14 g of UD1-FA, 1.08 g of TWEEN 80, and 0.08 g of HPMCAS were dissolved in 485 mL of THF. The solution was spray dried onto a mixture of 7.20 g of AVICEL PH101, 7.20 g of lactose DT, and 3.0 g of crospovidone using fluidized bed granulation (Vector Laboratory Micro Fluid Bed) equipment. The granules were passed through a #60 mesh screen to obtain mixture of fine powder and small granules. 1.28 g of this powder was thoroughly blended with 0.11 g of AVICEL PH101, 0.08 g of crospovidone, 0.19 g of corn starch, 0.03 g of CAB-O-SIL, 0.07 g of sodium lauryl sulfate, 0.75 g of anhydrous potassium carbonate, 0.25 g of anhydrous potassium bicarbonate, and 0.01 g of magnesium stearate. The resulting mixture was compressed into tablets using SC-2 single station tablet press from Key International; each tablet had hardness of 8-12 Kp. Each tablet weighed 555 mg and contained 100 mg of UD1-FA.

### Example 11

0.51 g of HPMC E3 and 0.41 g of sodium lauryl sulfate were dissolved in 101.1 g of water. 18.0 g of UD1-FA was added to this solution to form a suspension. This suspension was milled for 1.5 hours using a bead mill (Dyno-Mill, Glenn Mills Inc.). 1.0 g of TWEEN 80 was dissolved in 67.0 g of the prepared nanosuspension. The resulting nanosuspension was then spray dried onto a mixture of 6.0 g of AVICEL PH101, 6.0 g of lactose, 2.8 g of crospovidone, and 2.0 g of pregelatinized starch using a fluidized bed granulation (Vector Laboratory Micro Fluid Bed) equipment to obtain mixture of fine powder and small granules. 2.85 g of the powder was thoroughly blended with 1.04 g of AVICEL PH101, 0.52 g of pregelatinized starch, 0.52 g of Crospovidone, 1.50 g of anhydrous potassium carbonate, 0.50 g of anhydrous potassium bicarbonate, and 0.04 g of magnesium stearate. The resulting mixture was compressed into tablets using SC-2 single station tablet press from Key International; each tablet had hardness of 8-12 Kp. Each tablet weighed 697 mg and contained 100 mg of UD1-FA.

### Example 12

12.14 g of UD1-FA, 1.44 g of TWEEN 80, 1.44 g of Vitamin E TPGS, and 0.35 g of HPMCAS were dissolved in 485 mL of THF. The solution was spray dried onto a mixture of 7.20 g of Avicel PH101, 7.20 g of Lactose DT, and 3.0 g of crospovidone using fluidized bed granulation (Vector Laboratory Micro Fluid Bed) equipment. The granules were passed through a #60 mesh screen to obtain mixture of fine powder and small granules. 15.02 g of this powder was thoroughly blended with 1.42 g of AVICEL PH101, 0.88 g of crospovidone, 2.04 g of corn starch, 0.32 g of CAB-O-SIL, 0.79 g of sodium lauryl sulfate, 8.25 g of anhydrous potassium carbonate, 2.75 g of anhydrous potassium bicarbonate, and 0.16 g of magnesium stearate. The resulting mixture was compressed into tablets using SC-2 single station tablet press from Key International; each tablet had hardness of 8-12 Kp. Each tablet weighed 575 mg and contained 100 mg of UD1-FA.

### Example 13

0.90 g of the solid composition of Example 4 was thoroughly blended with 0.11 g of AC-DI-SOL, 0.11 g of pregelatinized starch, 0.11 g of AVICEL PH101, 0.08 g of corn starch, 0.02 g of CAB-O-SIL M5P, 0.08 g of sodium lauryl sulfate, 0.75 g of potassium carbonate, 0.25 g of potassium bicarbonate, and 0.01 g of magnesium stearate. The resulting mixture was compressed into tablets using SC-2 single station tablet press from Key International; each tablet had hardness of 8-12 Kp. Each tablet weighed 484 mg and contained 100 mg of UD1-FA.

### Example 14

90.0 g of TWEEN 80 and 27.0 g of PLASDONE K29/32 were dissolved in 9870.0 g of water. 900.0 g of UD1-FA was added to this solution to form a suspension. This suspension was milled using a bead mill (Dyno-Mill). The nanosuspension obtained was then passed through a #40 mesh screen. 10396.0 g of the nanosuspension was spray dried onto a mixture of 496.8 g of AVICEL PH101, 495.9 g of lactose DT, and 117.0 g of crospovidone using fluidized bed granulation equipment. The dried granules were then passed through a #40 mesh screen. 2126.7 g of the granulation was thoroughly blended with 55.8 g of AVICEL PH101, 55.8 g of lactose DT, and 11.7 g of magnesium stearate. The resulting mixture was compressed into tablets using "B" Type tablet press; each tablet had hardness of 8-12 Kp. Each tablet weighed 250 mg and contained 100 mg of UD1-FA.

### Example 15

This is a bilayer tablet formulation prepared using the solid composition of Example 4. 1.80 g of the solid composition of Example 4 was thoroughly blended with 0.23 g of AC-DI-SOL, 0.23 g of pregelatinized starch, 0.23 g of AVICEL PH101, 0.15 g of corn starch, 0.03 g of CAB-O-SIL M5P, 0.15 g of sodium lauryl sulfate, and 0.02 g of magnesium stearate. This forms the blend for drug layer. 1.50 g of potassium carbonate, 0.50 g of potassium bicarbonate, 0.20 g of crospovidone, 0.20 g of AVICEL PH101, 0.40 g of corn starch, and 0.03 g of magnesium stearate were thoroughly blended. This forms the blend for carbonate layer. Both the drug-containing blend and the carbonate-containing blends were then compressed into bilayer tablets using SC-2 single station tablet press from Key International; each tablet had hardness of 8-12 Kp. Each bilayer tablet weighed 567 mg and contained 100 mg of UD1-FA.

### Example 16

0.24 g of TWEEN 80 and 0.06 g of HPMC E3 LV were dissolved in 2 mL of water. UD1-FA was milled with mortar and pestle and passed through a #60 mesh screen. 6.39 g of UD1-FA was weighed and mixed with 1.11 g of AVICEL PH101, 1.11 g of lactose monohydrate and 0.73 g of AC-DI-SOL in a blender. The HPMC/TWEEN 80 solution was then added to the blender while mixing for 1-2 minutes. Additional water was added to complete the granulation. The wet granulation was dried in oven at 50 °C until dry. The dried granules were passed through a #30 mesh screen and mixed with 0.6 g AC-DI-SOL, 0.6 g pregelatinized starch and 1.11g AVICEL PH 101 for 15 minutes. 0.06 g of magnesium stearate was added and mixed for another 5 minutes. The resulting mixture was then filled in Swedish orange opaque capsules. Each capsule weighed 190 mg and contained 100 mg of UD1-FA.

### Example 17

UD1-FA was milled with mortar and pestle and passed through a #60 mesh screen. 6.39 g of UD1-FA was weighed and mixed with 2.30 g of AVICEL PH101, 0.72 g of AC-DI-SOL, 0.3 g of sodium lauryl sulfate, 0.06 g colloidal silicone dioxide, 0.6 g of pregelatinized starch, and 0.04 g of magnesium stearate in a blender. The mix was compressed into tablets (slugs) . The tablets were milled using CoMil equipped with a #050R screen. The milled material was passed through a #30 mesh screen and a #60 mesh screen. Material retained on the #30 mesh screen was milled again through Comil with a #032R screen and passed through a #30 mesh screen and a #60 mesh screen. All material passed through the 60 mesh screen was slugged again and milled, as previously. All milled and screened material (dry granulation) was mixed with 0.6 g AVICEL PH101, 0.36 g of AC-DI-SOL, 0.6 g of pregelatinized starch, and 0.03 g of magnesium stearate in a blender for 15 minutes. The resulting mixture was then filled in Swedish orange opaque capsules. Each capsule weighed 380 mg and contained 200 mg of UD1-FA.

### Example 18

The products of Examples 1 through 17 were each analyzed for in vivo bioavailability using dogs (male, beagle dogs (n=3), weighing 6.5-9.0 kg) and/or rats (n=3, weighing between 300-400 g). The products tested in rats were either administered in microcapsules (PCcaps, Capsugel, Greenwood, SC, USA) or as powder blend. The dose was administered orally to animals in the fasted state (where food was withheld overnight). Following dosing, blood samples for pharmacokinetic evaluation were collected from each animal at predose (t=0), and at 0.5, 1, 2, 3, 4, 6, 8, 12, and 24 hours following dosing. Blood was collected into lithium-hepranized tubes. After each time point, all blood samples were collected, processed, and frozen at about -70 °C.

The concentrations of the compound in rat and/or dog plasma were determined by a LC-MS/MS assay following a protein precipitation step with acetonitrile. Pharmacokinetic analysis was performed using the WinNonlin™ software program (Pharsight, Inc., Mountain View, Calif.). The area under the plasma concentration-time curve (AUC₀₋ₜ) is calculated from the first time point (0 min) up to the last time point with measurable drug concentration. The AUC_{0-inf} was calculated as the sum of AUC₀₋ₜ and Cpred/λz, where Cpred was the predicted concentration at the time of the last quantifiable concentration.

The results of a pharmacokinetic analysis of the solid compositions of Examples 1-13 in rats are shown below in Table 3. The results of a pharmacokinetic analysis of the solid compositions of Examples 3, 4, 6, 7, 11, 14 -17 in dogs are shown below in Table 4.

**Table 3**

| Example | Dose (mg/kg) | Cₘₐₓ (ng/mL) | AUC₀₋ₜ (hr^{∗}ng/mL) |
|---|---|---|---|
| 1 | 10 | 4150 | 40538 |
| 2 | 10 | 6695 | 41256 |
| 3 | 10 | 5875 | 45216 |
| 4 | 10 | 6595 | 52680 |
| 5 | 10 | 4710 | 45986 |
| 6 | 10 | 12500 | 63024 |
| 7 | 10 | 13150 | 58717 |
| 8 | 10 | 16300 | 60884 |
| 9 | 13.1 | 19933 | 140085 |
| 10 | 10 | 21313 | 141501 |
| 11 | 10 | 12713 | 74548 |
| 12 | 10 | 19833 | 150244 |
| 13 | 10 | 13767 | 100724 |

**Table 4**

| Example | Dose (mg/kg) | Cₘₐₓ (ng/mL) | AUC₀₋ₜ (hr^{∗}ng/mL) |
|---|---|---|---|
| 3 | 10.7 | 5303 | 14448 |
| 4 | 10.4 | 4127 | 11724 |
| 6 | 9.8 | 6500 | 14797 |
| 7 | 10.1 | 6113 | 12645 |
| 11 | 9.2 | 7222 | 12697 |
| 14 | 12.6 | 5703 | 15516 |
| 15 | 9.2 | 7787 | 14203 |
| 16 | 9.5 | 2363 | 6558 |
| 17 | 10.0 | 431 | 1985 |

## Claims

1. A solid composition comprising granules that are the product of a wet granulation process, wherein the granules comprise {2-[3-cyclohexyl-3-(*trans-*4-propoxycyclohexyl)-ureido]-thiazol-5-ylsulfanyl}-acetic acid, a binder, and a water-soluble surfactant
wherein the binder comprises polyvinylpyrrolidone.

2. The solid composition of claim 1, wherein the weight/weight ratio of {2-[3-cyclohexyl-3-(*trans*-4-propoxy-cyclohexyl)-ureido]-thiazol-5-ylsulfanyl} acetic acid to the water-soluble surfactant in the solid composition ranges from 10:1 to 100:1, or 15:1 to 60:1, or from 18:1 to 50:1, or from 22:1 to 40:1, or from 27:1 to 35:1; and
the weight/weight ratio of {2-[3-cyclohexyl-3-(*trans*-4-propoxy-cyclohexyl)-ureido]-thiazol-5-ylsulfanyl} acetic acid to the binder in the solid composition ranges from 25:1 to 400:1, or from 35:1 to 300:1, or from 50:1 to 250:1, or from 65:1 to 200:1, or from 75:1 to 150:1.

3. The solid composition of claim 1, wherein at least 85% of the particles of {2-[3-cyclohexyl-3-(*trans*-4-propoxy-cyclohexyl)-ureido]-thiazol-5-ylsulfanyl}-acetic acid used in the wet granulation process have a particle size between 0.4 µm and 6 µm.

4. The solid composition of any one of claims 1 or 2, wherein at least 80% of the granules by weight have a particle size that is between 1 µm and 1 mm.

5. The solid composition of any one of claims 1, 2 or 4, wherein at least 80% of the granules by weight have a particle size that is between 1 µm and 500 µm.

6. The solid composition of any one of claims 1 to 5, wherein the water-soluble surfactant is a sulfuric acid alkyl ester salt, a bile acid salt, a propylene glycol fatty acid mono- or diester, a polyethylene glycol fatty acid ester, a polyoxyethylene sorbitan fatty acid ester, a polyoxyethylene-polyoxypropylene copolymer or block copolymer surfactant, a polyoxyethylene derivative of a tocopherol or a tocotrienol, a polyoxyethylene derivative of a natural oil or wax, or a sorbitan fatty acid ester, or a mixture thereof.

7. The solid composition of claim 6, wherein the water-soluble surfactant is a sulfuric acid alkyl ester salt, a polyoxyethylene sorbitan fatty acid ester, or a polyoxyethylene derivative of a tocopherol or a tocotrienol, or a mixture thereof.

8. The solid composition of claim 7, wherein the water-soluble surfactant is sodium lauryl sulfate, polysorbate 80, or d-alpha-tocopheryl polyethylene glycol succinate, or a mixture thereof.

9. The solid composition of any one of claims 1 to 8, wherein the solid composition further comprises a pharmaceutically acceptable carrier or diluent, or a mixture thereof.

10. The solid composition of any one of claims 1 to 9, wherein the solid composition is in the form of a powder or capsule or tablet.

11. The solid composition of any one of claims 1 to 10, wherein the solid composition comprises crystalline {2-[3-cyclohexyl-3-(*trans*-4-propoxy-cyclohexyl)-ureido]-thiazol-5-ylsulfanyl}-acetic acid.

12. A method of making granules for use in preparation of a solid composition, wherein the method comprises the steps of:
a) mixing {2-[3-cyclohexyl-3-(*trans*-4-propoxy-cyclohexyl)-ureido]thiazol-5-ylsulfanyl}-acetic acid, a binder, and a water-soluble surfactant in the presence of a solvent to form a solution or suspension, wherein the binder comprises polyvinylpyrrolidone;
and
b) removing the solvent from the solution or suspension to form granules.

13. The method of claim 12, further comprising:
c) forming the granules into a tablet, or
c) encapsulating the granules, or
c) packaging the granules into a sachet.

14. A solid composition of claims 1 to 11 for use in a method of treating type 2 diabetes or type 1 diabetes.

15. A solid composition of claims 1 to 11 for use in a method of lowering blood glucose concentration in a human or activating glucokinase in a human or increasing hepatic glucose use in a human.

## Patentansprüche

1. Feste Zusammensetzung, die Granulate umfasst, die das Produkt eines Nassgranulationsvorgangs sind, wobei die Granulate {2-[3-Cyclohexyl-3-(*trans-*4-propoxycyclohexyl)-ureido]-thiazol-5-ylsulfanyl}-Essigsäure, ein Bindemittel und ein wasserlösliches Tensid umfassen,
wobei das Bindemittel Polyvinylpyrrolidon umfasst.

2. Feste Zusammensetzung nach Anspruch 1, wobei das Gewicht/Gewichtverhältnis von {2-[3-Cyclohexyl-3-(*trans*-4-propoxycyclohexyl)-ureido]-thiazol-5-ylsulfanyl}-Essigsäure zu dem wasserlöslichen Tensid in der festen Zusammensetzung in einem Bereich von 10:1 bis 100:1 oder 15:1 bis 60:1 oder von 18:1 bis 50:1 oder von 22:1 bis 40:1 oder von 27:1 bis 35:1 liegt; und
das Gewicht/Gewichtverhältnis von {2-[3-Cyclohexyl-3-(*trans*-4-propoxycyclohexyl)-ureido]-thiazol-5-ylsulfanyl}-Essigsäure zu dem Bindemittel in der festen Zusammensetzung in einem Bereich von 25:1 bis 400:1 oder von 35:1 bis 300:1 oder von 50:1 bis 250:1 oder von 65:1 bis 200:1 oder von 75:1 bis 150:1 liegt.

3. Feste Zusammensetzung nach Anspruch 1, wobei wenigstens 85 % der Partikel von {2-[3-Cyclohexyl-3-(*trans*-4-propoxycyclohexyl)-ureido]-thiazol-5-ylsulfanyl}-Essigsäure, die in dem Nassgranulationsvorgang verwendet werden, Partikelgrößen zwischen 0,4 µm und 6 µm aufweisen.

4. Feste Zusammensetzung nach einem der Ansprüche 1 oder 2, wobei wenigstens 80 Gew.-% der Granulate eine Partikelgröße zwischen 1 µm und 1 mm aufweisen.

5. Feste Zusammensetzung nach einem der Ansprüche 1, 2 oder 4, wobei wenigstens 80 Gew.-% der Granulate eine Partikelgröße zwischen 1 µm und 500 µm aufweisen.

6. Feste Zusammensetzung nach einem der Ansprüche 1 bis 5, wobei das wasserlösliche Tensid ein Schwefelsäurealkylestersalz, ein Gallensäuresalz, ein Propylenglykolfettsäuremono- oder -diester, ein Polyethylenglykolfettsäureester, ein Polyoxyethylensorbitanfettsäureester, ein Polyoxyethylen-Polyoxypropylen-Copolymer oder -Blockcopolymer-Tensid, ein Polyoxyethylenderivat eines Tocopherols oder eines Tocotrienols, ein Polyoxyethylenderivat eines natürlichen Öls oder Wachses oder ein Sorbitanfettsäureester oder ein Gemisch davon ist.

7. Feste Zusammensetzung nach Anspruch 6, wobei das wasserlösliche Tensid ein Schwefelsäurealkylestersalz, ein Polyoxyethylensorbitanfettsäureester oder ein Polyoxyethylenderivat eines Tocopherols oder eines Tocotrienols oder ein Gemisch davon ist.

8. Feste Zusammensetzung nach Anspruch 7, wobei das wasserlösliche Tensid Natriumlaurylsulfat, Polysorbat 80 oder d-alpha-Tocopherylpolyethylenglykolsuccinat oder ein Gemisch davon ist.

9. Feste Zusammensetzung nach einem der Ansprüche 1 bis 8, wobei die feste Zusammensetzung ferner einen pharmazeutisch unbedenklichen Träger oder ein pharmazeutisch unbedenkliches Verdünnungsmittel oder ein Gemisch davon umfasst.

10. Feste Zusammensetzung nach einem der Ansprüche 1 bis 9, wobei die feste Zusammensetzung in der Form eines Pulvers oder einer Kapsel oder einer Tablette vorliegt.

11. Feste Zusammensetzung nach einem der Ansprüche 1 bis 10, wobei die feste Zusammensetzung kristalline {2-[3-Cyclohexyl-3-(*trans*-4-propoxycyclohexyl)-ureido]-thiazol-5-ylsulfanyl}-Essigsäure umfasst.

12. Verfahren zum Fertigen von Granulaten zur Verwendung bei der Herstellung einer festen Zusammensetzung, wobei das Verfahren die Schritte umfasst:
a) Mischen von {2-[3-Cyclohexyl-3-(*trans*-4-propoxycyclohexyl)-ureido]-thiazol-5-ylsulfanyl}-Essigsäure, einem Bindemittel und einem wasserlöslichen Tensid in der Gegenwart eines Lösungsmittels, um eine Lösung oder eine Suspension auszubilden, wobei das Bindemittel Polyvinylpyrrolidon umfasst;
und
b) Entfernen des Lösungsmittels aus der Lösung oder der Suspension, um Granulate auszubilden.

13. Verfahren nach Anspruch 12, das ferner umfasst:
c) Ausbilden der Granulate zu einer Tablette, oder
c) Einkapseln der Granulate, oder
c) Verpacken der Granulate in ein Päckchen.

14. Feste Zusammensetzung nach den Ansprüchen 1 bis 11 zur Verwendung in einem Verfahren zur Behandlung von Diabetes Typ 2 oder Diabetes Typ 1.

15. Feste Zusammensetzung nach den Ansprüchen 1 bis 11 zur Verwendung in einem Verfahren zum Senken der Blutglucosekonzentration bei einem Menschen oder zum Aktivieren der Glucokinase bei einem Menschen oder zum Erhöhen der Verwendung der hepatischen Glucose bei einem Menschen.

## Revendications

1. Composition solide comprenant des granules qui sont le produit d'un procédé de granulation humide, dans laquelle les granules comprennent {2-[3-cyclohexyl-3-(*trans-*4-propoxycyclohexyl)-uréido]-thiazol-5-ylsulfanyl}-acide acétique, un liant et un surfactant soluble dans l'eau
dans laquelle le liant comprend le polyvinylpyrrolidone.

2. Composition solide selon la revendication 1, dans laquelle le rapport poids/poids de {2-[3-cyclohexyl-3-(*trans-*4-propoxy-cyclohexyl)-uréido]-thiazol-5-ylsulfanyl}acide acétique au surfactant soluble dans l'eau dans la composition solide va de 10:1 à 100:1, ou de 15:1 à 60:1, ou de 18:1 à 50:1, ou de 22:1 à 40:1, ou de 27:1 à 35:1 ; et
le rapport poids/poids de {2-[3-cyclohexyl-3-(*trans*-4-propoxy-cyclohexyl)-uréido]-thiazol-5-ylsulfanyl}acide acétique au liant dans la composition solide va de 25:1 à 400:1, ou de 35:1 à 300:1, ou de 50:1 à 250:1, ou de 65:1 à 200:1, ou de 75:1 à 150:1.

3. Composition solide selon la revendication 1, dans laquelle au moins 85 % des particules de {2-[3-cyclohexyl-3-(*trans-*4-propoxy-cyclohexyl)-uréido]-thiazol-5-ylsulfanyl}-acide acétique utilisées dans le procédé de granulation humide ont une taille de particule comprise entre 0,4 µm et 6 µm.

4. Composition solide selon l'une quelconque des revendications 1 ou 2, dans laquelle au moins 80 % des granules en poids ont une taille de particule comprise entre 1 µm et 1 mm.

5. Composition solide selon l'une quelconque des revendications 1, 2 ou 4, dans laquelle au moins 80 % des granules en poids ont une taille de particule comprise entre 1 µm et 500 µm.

6. Composition solide selon l'une quelconque des revendications 1 à 5, dans laquelle le surfactant soluble dans l'eau est un sel d'ester alkylique de l'acide sulfurique, un sel d'acide biliaire, un mono- ou diester d'acide gras de propylène glycol, un ester d'acide gras de polyéthylène glycol, un ester d'acide gras polyoxyéthyléné de sorbitane, un surfactant de copolymère de polyoxyéthylène-polyoxypropylène ou de copolymère séquencé, un dérivé polyoxyéthyléné d'un tocophérol ou d'un tocotriénol, un dérivé polyoxyéthyléné d'une huile ou cire naturelle, ou un ester d'acide gras de sorbitane, ou un mélange de ceux-ci.

7. Composition solide selon la revendication 6, dans laquelle le surfactant soluble dans l'eau est un sel d'ester alkylique de l'acide sulfurique, un ester d'acide gras polyoxyéthyléné de sorbitane, ou un dérivé polyoxyéthyléné d'un tocophérol ou d'un tocotriénol, ou un mélange de ceux-ci.

8. Composition solide selon la revendication 7, dans laquelle le surfactant soluble dans l'eau est le laurylsulfate de sodium, le polysorbate 80, ou le succinate de d-alpha-tocophéryl polyéthylène glycol, ou un mélange de ceux-ci.

9. Composition solide selon l'une quelconque des revendications 1 à 8, la composition solide comprenant en outre un excipient ou diluant de qualité pharmaceutique, ou un mélange de ceux-ci.

10. Composition solide selon l'une quelconque des revendications 1 à 9, la composition solide étant sous forme de poudre, de capsule ou de comprimé.

11. Composition solide selon l'une quelconque des revendications 1 à 10, la composition solide comprenant du {2-[3-cyclohexyl-3-(*trans*-4-propoxy-cyclohexyl)-uréido]-thiazol-5-ylsulfanyl}-acide acétique cristallin.

12. Procédé de fabrication de granules devant être utilisées dans la préparation d'une composition solide, le procédé comprenant les étapes de :
a) mélange du {2-[3-cyclohexyl-3-(*trans*-4-propoxy-cyclohexyl)-uréido]thiazol-5-ylsulfanyl}-acide acétique, d'un liant et d'un surfactant soluble dans l'eau en présence d'un solvant pour former une solution ou une suspension, dans lequel le liant comprend le polyvinylpyrrolidone ; et
b) d'enlèvement du solvant de la solution ou suspension pour former des granules.

13. Procédé selon la revendication 12, comprenant en outre :
c) le formage d'un comprimé à partir des granules, ou
c) l'encapsulation des granules, ou
c) le conditionnement des granules dans un sachet.

14. Composition solide selon les revendications 1 à 11 devant être utilisée dans un procédé de traitement du diabète de type 2 ou du diabète de type 1.

15. Composition solide selon les revendications 1 à 11, devant être utilisée dans un procédé d'abaissement de la glycémie chez un sujet humain ou d'activation de la glucokinase chez un sujet humain ou d'augmentation de l'utilisation du glucose hépatique chez un sujet humain.
